(19) Europäisches Patentamt
European Patent Office
Office européen des brevets

(11) Veröffentlichungsnummer: **0 000 718**
**B1**

(12) **EUROPÄISCHE PATENTSCHRIFT**

(45) Veröffentlichungstag der Patentschrift: **24.03.82**

(21) Anmeldenummer: **78100471.8**

(22) Anmeldetag: **21.07.78**

(51) Int. Cl.³: **C 07 D 487/04,**
A 61 K 31/505
//C07C101/18, C07D239/78,
(C07D487/04, 239/00,
235/00)

# (54) Neue Chinazolinderivate, Verfahren zu ihrer Herstellung, pharmazeutische Präparate und deren Herstellung.

(30) Priorität: **25.07.77 LU 77829**
**26.05.78 CH 5776/78**

(43) Veröffentlichungstag der Anmeldung:
**21.02.79 Patentblatt 79/4**

(45) Bekanntmachung des Hinweises auf die Patenterteilung:
**24.03.82 Patentblatt 82/12**

(84) Benannte Vertragsstaaten:
**BE CH DE FR LU NL SE**

(56) Entgegenhaltungen:
**DE - A - 1 445 906**
**DE - A - 2 305 575**

(73) Patentinhaber: **F. HOFFMANN-LA ROCHE & CO. Aktiengesellschaft**
**CH-4002 Basel (CH)**

(72) Erfinder: **Chodnekar, Madhukar Subraya, Dr.**
**Rebhaldenstrasse 6**
**CH-4411 Seltisberg (CH)**
Erfinder: **Kaiser, Ado, Dr.**
**Hirsweg 3**
**CH-4415 Lausen (CH)**

(74) Vertreter: **Lederer, Franz, Dr. et al,**
**Patentanwälte Lederer, Meyer Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 000 718 B1

# Neue Chinazolinderivate, Verfahren zu ihrer Herstellung, pharmazeutische Präparate und deren Herstellung

Die vorliegende Erfindung betrifft neue tricyclische Verbindungen, nämlich Imidazo-chinazoline der Formel

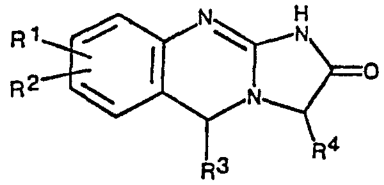
I

worin $R^1$ und $R^2$ Wasserstoff, $C_{1-6}$-Alkyl, Hydroxy, $C_{1-6}$-Alkoxy, Hydroxy-$C_{1-6}$-alkyl, $C_{1-6}$-Alkoxy-$C_{1-6}$-alkyl, Halogen, Phenyl, Phenoxy, Amino, $C_{1-6}$-Alkylamino oder di-$C_{1-6}$-Alkylamino, und $R^1$ und $R^2$ an benachbarten Kohlenstoffatomen auch gemeinsam Methylendioxy; $R^3$ Wasserstoff, $C_{1-6}$-Alkyl oder Phenyl; und $R^4$ $C_{1-6}$-Alkyl, Hydroxy-$C_{1-6}$-alkyl, $C_{1-6}$-Alkoxy-$C_{1-6}$-alkyl, Aryl-$C_{1-6}$-alkyl oder Aryl bedeuten,
deren Tautomere und Salze solcher Verbindungen.
Im Gegensatz zu den aus der DE—A—2305575 bekannten Imidazochinazolinen enthalten die vorliegenden Imidazochinazoline einen Substituenten in 3-Stellung.

Alkyl- und Alkoxyreste enthalten vorzugsweise 1—4 C-Atome. Alkylreste können geradkettig oder verzweigt sein. Beispiele von Alkylresten sind: Methyl, Aethyl, Propyl, Isopropyl, Butyl, Isobutyl, Pentyl und Hexyl. Aryl bedeutet insbesondere Phenyl oder durch Halogen, $C_{1-6}$-Alkyl, Hydroxy und/oder $C_{1-6}$-Alkoxy substituiertes Phenyl.

Unter den Verbindungen der Formel I sind
D-6-Chlor-1,5-dihydro-3-methyl-imidazo[2,1-b]chinazolin-2(3H)-on und deren Salze bevorzugt.

Beispiele von Verbindungen der Formel I sind
D-1,5-Dihydro-3,6-dimethyl-imidazo[2,1-b]chinazolin-2(3H)-on,
D-7-Brom-1,5-dihydro-3-methyl-imidazo[2,1-b]chinazolin-2(3H)-on,
L-6-Chlor-1,5-dihydro-3-hydroxymethyl-imidazo[2,1-b]chinazolin-2(3H)-on,
L-6-Chlor-1,5-dihydro-3-phenyl-imidazo[2,1-b]chinazolin-2(3H)-on,
L-6-Chlor-1,5-dihydro-3-isobutyl-imidazo[2,1-b]chinazolin-2(3H)-on,
L-3-Benzyl-6-chlor-1,5-dihydro-imidazo[2,1-b]chinazolin-2(3H)-on
und deren Salze.

Die Erfindung betrifft weiterhin ein Verfahren zur Herstellung der genannten Verbindungen sowie pharmazeutische Präparate auf der Basis der genannten Verbindungen.

Die Verbindungen der Formel I können in verschiedenen tautomeren Formen vorliegen. Die Erfindung beschränkt sich daher nicht auf Verbindungen der oben dargestellten Formel I, sondern umfasst auch die Tautomeren, beispielsweise solche der Formel

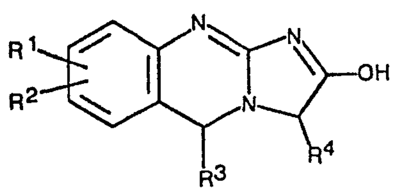
Ia

und

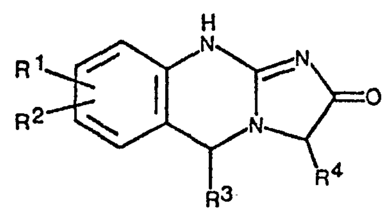
Ib

Die Verbindungen der Formel I und deren Tautomeren, z.B. Ia und Ib können weiterhin in Form von Racematen oder in optisch aktiver Form vorliegen, wobei alle diese Formen Gegenstand der Erfindung sind.

Beispiele physiologisch verträglicher Salze sind Mineralsäuresalze, wie Hydrochloride, Hydrobromide, Sulfate und Phosphate; Salze organischer Sulfonsäuren, wie Alkylsulfate und Arylsulfonate; und Carbonsäuresalze, wie Succinate, Citrate, Tartrate und Maleate.

Die Verbindungen der Formel I und deren Tautomeren können erfindungsgemäss dadurch hergestellt werden, dass man

a) eine Verbindung der Formel

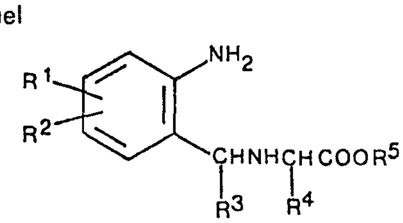
II

worin $R^1$—$R^4$ die obige Bedeutung haben und $R^5$ $C_{1-6}$-Alkyl darstellt,

mit Bromcyan umsetzt, oder
b) eine Verbindung der Formel

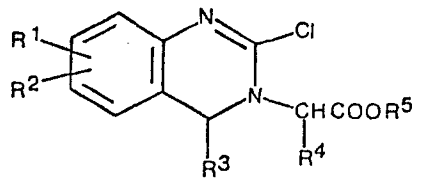

III

worin $R^1$—$R^5$ die obige Bedeutung haben,
mit Ammoniak behandelt.

Die Umsetzung einer Verbindung der Formel II mit Brom-cyan wird zweckmässig unter Erwärmen in einem Lösungsmittel wie einem niederen Alkohol, z.B. Aethanol durchgeführt. Die Umsetzung einer Verbindung der Formel III mit Ammoniak wird zweckmässig unter Erwärmen in einem Lösungsmittel, wie einem niederen Alkohol, z.B. Aethanol, und Wasser, durchgeführt.

Eine Verbindung der Formel I, worin $R^1$ und/oder $R^2$ Wasserstoff ist, kann in an sich bekannter Weise halogeniert werden. So kann man z.B. eine Lösung einer in den Stellungen 6, 7, 8 und 9 unsubstituierten Verbindung in Essigsäure mit Brom zur 7-Bromverbindung umsetzen.

Die Verbindungen der Formel I, worin $R^1$ und $R^2$ von einer gegebenenfalls alkylierten Aminogruppe verschieden sind, können nach dem nachstehend wiedergegebenen Formelschema I hergestellt werden, worin Y Chlor oder Brom darstellt, $R^{11}$ und $R^{21}$ die gleichen Bedeutungen haben wie $R^1$ und $R^2$ mit Ausnahme von gegebenenfalls alkyliertem Amino und $R^3$ und $R^4$ obige Bedeutung haben.

Die Verbindungen der Formel I können ferner nach dem nachstehend wiedergegebenen Formelschema II hergestellt werden, worin Z Sauerstoff oder Schwefel, M Ammonium, Kalium oder Natrium darstellen und die restlichen Symbole obige Bedeutung haben.

Formelschema I

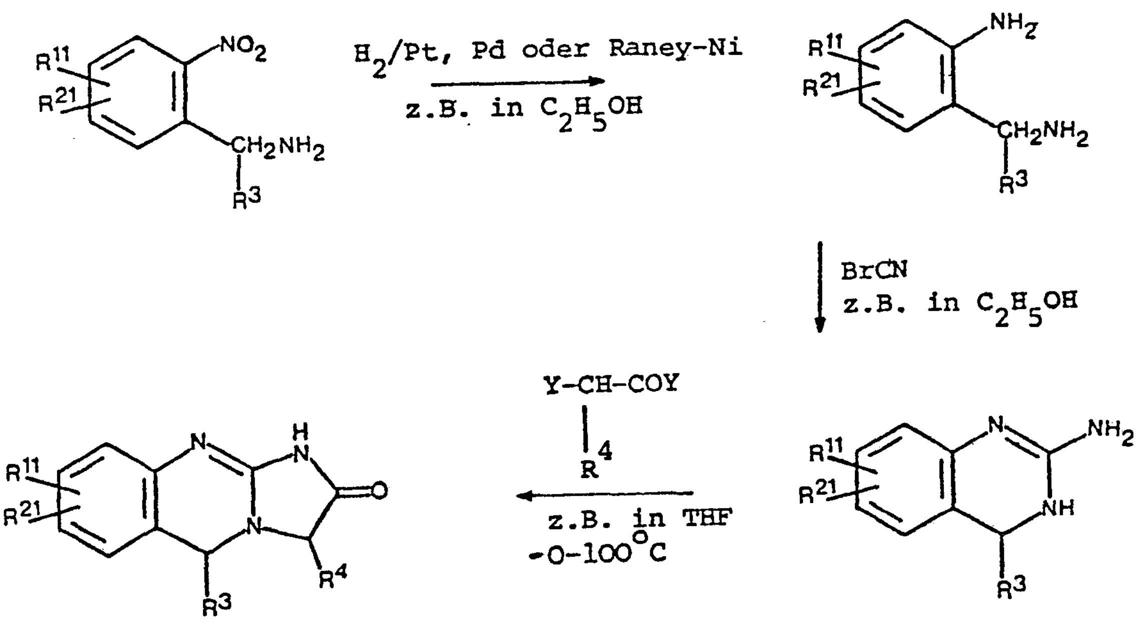

Formelschema II

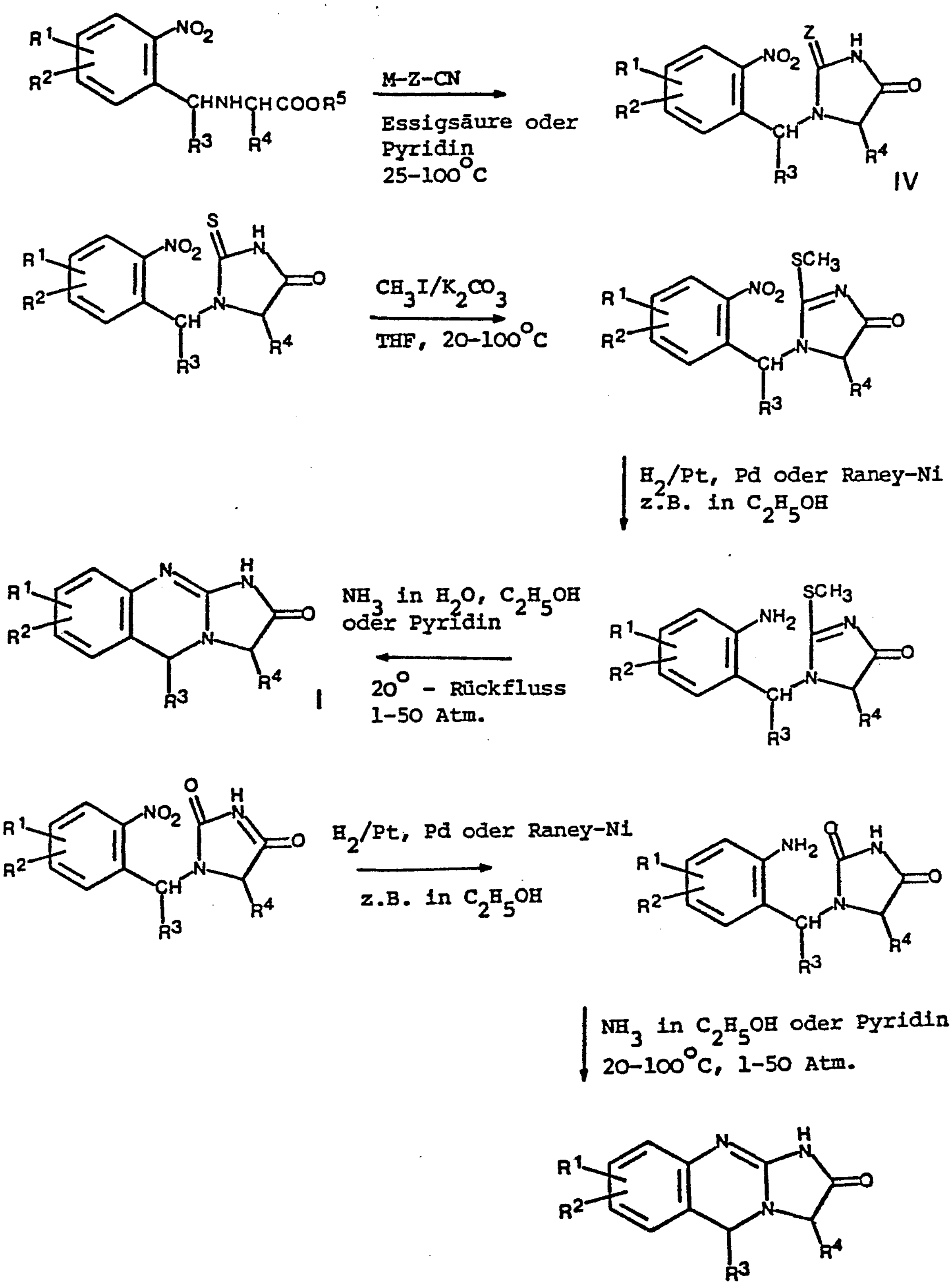


Die Verbindungen der Formel II sind neu.

Die Ausgangsverbindungen der Formeln II und III können nach dem nachstehend wiedergegebenen Formelschema III, worin X Halogen darstellt und die restlichen Symbole obige Bedeutung haben, bzw. in Analogie zu den in den Beispielen angegebenen Methoden hergestellt werden.

Formelschema III

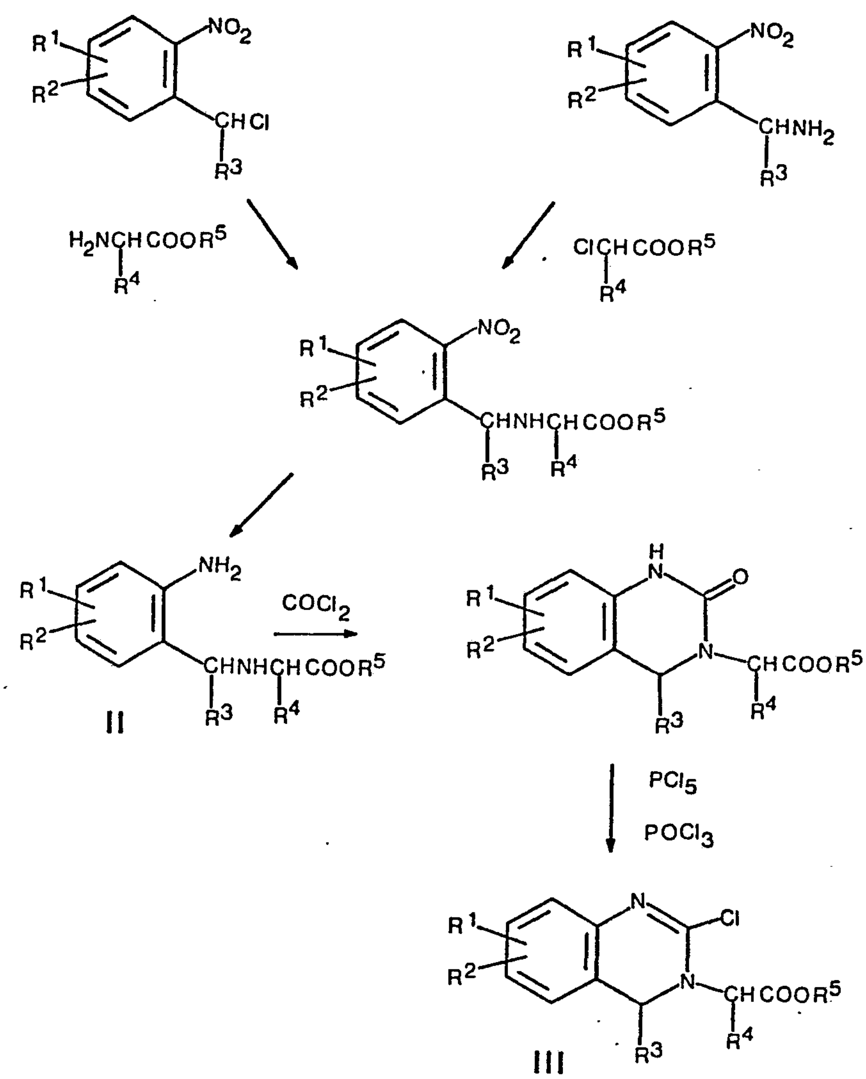


II

III

Die Verbindungen der Formel I, deren Tautomere und physiologisch verträgliche Salze solcher Verbindungen sollen als Heilmittel Verwendung finden. Sie hemmen z.B. die Aggregation der Blutplättchen und können daher zur Verhütung von Thrombosen verwendet werden. Ausserdem sind sie kreislaufwirksam. So können sie auf Grund ihrer positiv inotropen Wirkung ohne wesentliche Tachycardie zur Behandlung und Prophylaxe von Herzversagen und Herzschwäche verwendet werden.

Die Verbindungen der Formel I und deren Tautomere können als Heilmittel z.B. in Form pharmazeutischer Präparate Verwendung finden, welche sie oder ihre Salze in Mischung mit einem für die enterale, perkutane oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline®, usw. enthalten. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierunga-, Netz- oder Emulgiermittel, Salze zur Veränderung des osmotischen Druckes oder Puffer. Sie können auch noch andere therapeutisch wertvolle Stoffe enthalten. Die orale Verabreichung der erfindungsgemässen Verbindungen ist bevorzugt. Für den Erwachsenen kommen eine orale Tagesdosis von 0,5 bis 30 mg/kg und eine parenterale Tagesdosis von 0,05 bis 10 mg/kg in Frage.

Die aggregationshemmende Wirkung wurde nach der Aggregometer-Methode von BORN [Nature *194*, 927 (1962)] und MICHAL und BORN [Nature *231*, 220 (1971)] nachgewiesen. Die maximale Aggregationsgeschwindigkeit wurde als Versuchsparameter genommen und die effektive Konzentration ($EC_{50}$) aus Dosis-Wirkungskurven ermittelt.

® Warenzeichen

Menschliches Plasma wurde aus mit Citrat (10,6 mM) zersetztem venösem Blut durch Zentrifugieren erhalten. 0,18 ml Plasma wurden mit 10 μl wässriger Suspension der Testverbindungen versetzt, 10 Minuten bei 37°C inkubiert, worauf die Aggregation durch Zusatz von 10 μl Collagen-Fibril-Suspension eingeleitet wurde.

Kaninchen-Plasma wurde aus mit Citrat (9 mM) zersetztem arteriellem Blut durch Zentrifugieren erhalten. 1 ml Plasma wurde mit 10 μl Test-Lösung versetzt und 1 Minute bei 37°C inkubiert, worauf 8 μl Collagen-Fibril-Suspension oder 10 μl Adenosindiphosphat (ADP) in $10^{-4}$ M Kochsalzlösung zugesetzt wurden. Als Kontrollwert diente mit Dimethylsulfoxid inkubiertes Plasma.

Die Resultate sind in der nachstehenden Tabelle I wiedergegeben.

Die positiv inotrope Wirkung wurde nach oraler Gabe der Prüfsubstanzen an wachen Schäferhunden gemessen. Die Tiere sind zu diesem Zweck mit einem implantierten Druck-Telemetrie-System ausgerüstet, wobei der Druckaufnehmer im linken Ventrikel fixiert ist. Der linksventrikuläre Druck wird über den implantierten Radiosender aus dem Tiergesendet und über ein geeignetes Antennen- und Empfängersystem empfangen, demoduliert und verstärkt. Durch Differenzierung des ansteigenden Schenkels des linksventrikulären Druckes (LVP) wird die maximale Druckanstiegsgeschwindigkeit ($dLVP/dt_{max}$) errechnet, was als Kontraktilitätsparameter gilt. Gleichzeitig wird die Herzfrequenz über einen Cardiotachographen aufgezeichnet. Unter Inotropie werden die prozentuale Veränderung (Δ%) von $dLVP/dt_{max}$ und die Wirkungsdauer in Minuten (Min) angegeben. Unter Tachycardie werden die prozentualen Veränderungen der Herzfrequenz (Δ%) nach Gabe der Prüfsubstanz und die Wirkungsdauer in Minuten (Min) angegeben. Die Resultate sind in der nachstehenden Tabelle II wiedergegeben.

TABELLE I

Collagen- und ADP-induzierte Blutplättchenaggregation

| Verbindung | Kaninchen-Plasma Collagen EC 50 μM | Kaninchen-Plasma ADP EC 50 μM | menschliches Plasma Collagen EC 50 μM |
|---|---|---|---|
| D-1,5-Dihydro-3,9-dimethyl-imidazo[2,1-b]chinazolin-2(3H)-on-hydrochlorid | 3,0 | 32 | 26 |
| L-1,5-Dihydro-3,9-dimethyl-imidazo[2,1-b]chinazolin-2(3H)-on-hydrochlorid | 18 | 60 | 49 |
| D-1,5-Dihydro-3,7-dimethyl-imidazo[2,1-b]chinazolin-2(3H)-on-hydrochlorid | 3,1 | 19 | 3,4 |
| L-1,5-Dihydro-3,7-dimethyl-imidazo[2,1-b]chinazolin-2(3H)-on-hydrochlorid | 22 | 77 | 33 |
| D-1,5-Dihydro-3,6-dimethyl-imidazo[2,1-b]chinazolin-2(3H)-on-hydrochlorid | 0,19 | 2,2 | 2,3 |
| L-1,5-Dihydro-3,6-dimethyl-imidazo[2,1-b]chinazolin-2(3H)-on-hydrochlorid | 0,93 | 11 | 6,2 |
| L-1,5-Dihydro-3-hydroxymethyl-6-methyl-imidazo-[2,1-b]chinazolin-2-(3H)-on-hydrochlorid | – | – | 14 |

TABELLE II

| Verbindung | Dosis mg/kg | Inotropie Δ% | Inotropie Min | Herzfrequenz Δ% | Herzfrequenz Min |
|---|---|---|---|---|---|
| D-6-Chlor-1,5-dihydro-3-methyl-imidazo[2,1-b]chinazolin-2(3H)-on-hydrochlorid | 5 | 93 | 145 | 28 | 115 |
| D-1,5-Dihydro-3,6-dimethyl-imidazo[2,1-b]chinazolin-2(3H)-on-hydrochlorid | 10 | 82 | 440 | 63 | 480 |
| L-1,5-Dihydro-/3,9-dimethyl-imidazo[2,1-b]chinazolin-2(3H)-on-hydrochlorid | 10 | 43 | 120 | 16 | 100 |

Die folgenden Beispiele erläutern die Erfindung. Die Temperaturen sind in °C angegeben.

Beispiel 1

Zu einer Lösung von 11,8 g N-(2-Amino-3-methylbenzyl)-L-alanin-äthylester in 30 ml Aethanol wurde bei Raumtemperature unter Rühren eine Lösung von 5,3 g Bromcyan in 10 ml Aethanol gegeben. Das Reaktionsgemisch wurde 1 Stunde zum Rückfluss erhitzt und dann unter vermindertem Druck zur Trockene eingedampft. Der Rückstand wurde mit 100 ml Wasser versetzt und durch Zusatz von 3 N Ammoniumhydroxyd unter Rühren alkalisch gestellt. Das Gemisch wurde dann weitere 30 Minuten gerührt und dreimal mit je 100 ml Methylenchlorid extrahiert. Die organischen Extrakte wurden zweimal mit je 150 ml Wasser gewaschen, über Natriumsulfat getrocknet und eingedampft. Der Rückstand wurde aus Aethanol umkristallisiert und lieferte L-1,5-Dihydro-3,9-dimethylimidazo[2,1-b]-chinazolin-2(3H)-on, Schmelzpunkt 259—261°, $[\alpha]_D + 15,5°$ (C = 1% in Methanol).

Durch Umkristallisation der so erhaltenen Base aus 1 N Salzsäure und Acetonitril (3:1) wurde das Hydrochlorid vom Schmelzpunkt 272—275° (Zers.) erhalten.

Das Ausgangsmaterial wurde wie folgt hergestellt:

Zu einer Lösung von 91,8 g L-Alanin-äthylester-hydrochlorid in 300 ml absolutem Aethanol wurde innerhalb von 30 Minuten eine Lösung von 120 Triäthylamin in 200 ml absolutem Aethanol getropft. Das Reaktionsgemisch wurde auf 60° erwärmt, wobei eine klare Lösung entstand. Zu dieser Lösung wurde innerhalb 1 Stunde eine Lösung von 55,5 g 3-(Chlormethyl)-2-nitrotoluol in 300 ml absolutem Aethanol getropft. Danach wurde die Temperatur auf 80° erhöht und das Reaktionsgemisch über Nacht bei dieser Temperatur gerührt. Danach wurde unter vermindertem Druck zur Trockene eingedampft und der Rückstand in 600 ml Wasser gelöst. Die Lösung wurde dreimal mit Methylenchlorid extrahiert und die Extrakte wurden nacheinander mit Wasser und mit gesättigter Natriumchloridlösung gewaschen, getrocknet und eingedampft. Das so erhaltene Rohprodukt wurde durch Chromatographie an Kieselgel mit Methylenchlorid/5% Methanol als Laufmittel gereinigt. Man erhielt N-(3-Methyl-2-nitrobenzyl)-L-alanin-äthylester als gelbes Oel, $[\alpha]_D - 36°$ (C = 1% in Methanol).

Eine Lösung von 26,6 g N-(3-Methyl-2-nitrobenzyl)-L-alanin-äthylester in 100 ml absolutem Aethanol wurde in Gegenwart von 2 g 10%igem Pd/C hydriert. In 5 Stunden wurden 6,7 l Wasserstoff aufgenommen. Nach beendeter Hydrierung wurde von Katalysator abfiltriert und das Filtrat zur Trockene eingedampft. Man erhielt N-(2-Amino-3-methylbenzyl)-L-alanin-äthylester als gelbes Oel, $[\alpha]_D - 52,6°$ (C = 1% in Methanol).

Analog wurden folgende Verbindungen hergestellt:

aus 3-(Chlormethyl)-2-nitrotoluol und D-Alanin-äthylester-hydrochlorid der N-(3-methyl-2-nitrobenzyl)-D-alanin-äthylester, gelbes Oel, $[\alpha]_D + 31,4°$ (c = 1% in Methanol);

aus $\alpha^3$-Chlor-4-nitro-m-xylol und L-Alanin-äthylester-hydrochlorid der N-(5-Methyl-2-nitrobenzyl)-L-alanin-äthylester, rotes Oel, $[\alpha]_D - 12,6°$ (c = 1% in Methanol);

aus $\alpha^3$-Chlor-4-nitro-m-xylol und D-Alanin-äthylester-hydrochlorid der N-(5-Methyl-2-nitrobenzyl)-D-alanin-äthylester, rotes Oel, $[\alpha]_D + 11,4°$ (c = 1% in Methanol);

aus $\alpha^2$-Chlor-3-nitro-o-xylol und L-Alanin-äthylester-hydrochlorid der N-(2-Methyl-6-nitrobenzyl)-L-alanin-äthylester, rotes Oel, $[\alpha]_D + 35,8°$ (c = 1% in Methanol);

aus $\alpha^2$-Chlor-3-nitro-o-xylol und D-Alanin-äthylester-hydrochlorid der N-(2-Methyl-6-nitrobenzyl)-D-alanin-äthylester, rotes Oel, $[\alpha]_D - 34°$ (c = 1% in Methanol);

aus $\alpha^2$-Chlor-3-nitro-o-xylol und L-Serin-äthylester-hydrochlorid der N-(2-Methyl-6-nitrobenzyl)-L-serin-äthylester, rotes Oel, $n_D^{24} = 1,5474$;

aus $\alpha^2$-Chlor-3-nitro-o-xylol und D-$\alpha$-Phenylglycin-äthylester-hydrochlorid der N-(2-Methyl-6-nitrobenzyl)-D-$\alpha$-phenylglycin-äthylester, rotes Oel, $n_D^{24} = 1,5261$;

aus 2-Nitrobenzylchlorid und L-Alanin-äthylester-hydrochlorid der 2-Nitrobenzyl-L-alanin-äthylester, dunkelrotes Oel, $[\alpha]_D - 5,4°$ (c = 1% in Aethanol);

aus 2-Nitrobenzylchlorid und D-Alanin-äthylester-hydrochlorid der (2-Nitrobenzyl)-D-alanin-äthylester, rotes Oel, $[\alpha]_D + 5,4°$ (c = 1% in Aethanol);

aus N-3-Methyl-2-nitrobenzyl-D-alanin-äthylester der N-(2-Amino-3-methylbenzyl)-D-alanin-äthylester, hellgelbes Oel, $[\alpha]_D +51°$ (c = 1% in Methanol);

aus N-(5-Methyl-2-nitrobenzyl)-L-anilin-äthylester der N-(2-Amino-5-methylbenzyl)-L-alanin-äthylester, $[\alpha]_D - 45°$ (c = 1% in Methanol);

aus N-(5-Methyl-2-nitrobenzyl)-D-alanin-äthylester der N-(2-Amino-5-methylbenzyl)-D-alanin-äthylester, rotes Oel, $[\alpha]_D + 34,2°$ (c = 1% in Methanol);

aus N-(2-Methyl-6-nitrobenzyl)-L-alanin-äthylester der N-(2-Amino-6-methylbenzyl)-L-alanin-äthylester, gelbes Oel, $[\alpha]_D - 34,7°$ (c = 1% in Methanol);

aus N-(2-Methyl-6-nitrobenzyl)-D-alanin-äthylester der N-(2-Amino-6-methylbenzyl)-D-alanin-äthylester, rötliches Oel, $[\alpha]_D + 36,8°$ (c = 1% in Methanol);

aus N-(2-Methyl-6-nitrobenzyl)-L-serin-äthylester der N-(2-Amino-6-methylbenzyl)-L-serin-äthylester, rotes Oel, $n_D^{24} = 1,5468$;

aus N-(2-Methyl-6-nitrobenzyl)-D-$\alpha$-phenylglycin-äthylester der N-(2-Amino-6-methylbenzyl)-D-$\alpha$-phenylglycin-äthylester, gelbes Oel, $n_D^{24} = 1,5665$;

aus (2-Nitrobenzyl)-L-alanin-äthylester der 2-Aminobenzyl-L-alanin-äthylester, rotes Oel, $[\alpha]_D - 55,1°$ (c = 1% in Aethanol);

aus 2-Nitrobenzyl-D-alanin-äthylester der 2-Aminobenzyl-D-alanin-äthylester, dunkelrotes Oel, $[\alpha]_D + 57,2°$ (c = 1% in Aethanol).

### Beispiel 2

In Analogie zu Beispiel 1 wurde aus N-(2-Amino-3-methylbenzyl)-D-alanin-äthylester das D-1,5-Dihydro-3,9-dimethylimidazo[2,1-b]chinazolin-2(3H)-on-hydrochlorid erhalten. Schmelzpunkt 270—275° (Zers.). Die freie Base schmilzt bei 262—265°.

### Beispiel 3

In Analogie zu Beispiel 1 wurde aus N-(2-Amino-5-methylbenzyl)-L-alanin-äthylester das L-1,5-Dihydro-2,7-dimethyl-imidazo[2,1-b]chinazolin-2(3H)-on-hydrochlorid erhalten. Hellgelbe Kristalle, Schmelzpunkt 173—176°. Die freie Base schmilzt unter Zersetzung über 300°.

### Beispiel 4

In Analogie zu Beispiel 1 wurde aus N-(2-Amino-5-methylbenzyl)-D-alanin-äthylester das D-1,5-Dihydro-3,7-dimethyl-imidazo[2,1-b]chinazolin-2(3H)-on-hydrochlorid erhalten. Hellgelbe Kristalle, Schmelzpunkt 173—176° (Zers.). Die freie Base schmilzt unter Zersetzung bei 310—314°.

### Beispiel 5

In Analogie zu Beispiel 1 wurde aus N-(2-Amino-6-methylbenzyl)-L-alanin-äthylester das L-1,5-Dihydro-3,6-dimethyl-imidazo[2,1-b]chinazolin-2(3H)-on-hydrochlorid erhalten. Farblose Kristalle vom Schmelzpunkt 285—288° (Zers.). Die freie Base schmilzt oberhalb 340° unter Zersetzung.

### Beispiel 6

In Analogie zu Beispiele 1 wurde aus N-(2-Amino-6-methylbenzyl)-D-alanin-äthylester das D-1,5-Dihydro-3,6-dimethyl-imidazo[2,1-b]chinazolin-2(3H)-on-hydrochlorid erhalten. Hellgelbe Kristalle vom Schmelzpunkt 287—290° (Zers.). Die freie Base schmilzt oberhalb 340°.

### Beispiel 7

In Analogie zu Beispiel 1 wurde aus N-(2-Amino-6-methylbenzyl)-L-serin-äthylester das L-1,5-Dihydro-3-hydroxymethyl-6-methyl-imidazo[2,1-b]chinazolin-2(3H)-on-hydrochlorid erhalten. Gelbe Kristalle vom Schmelzpunkt 320—325° (Zers.).

### Beispiel 8

In Analogie zu Beispiel 1 wurde aus N-(2-Amino-6-methylbenzyl)-D-$\alpha$-phenylglycin-äthylester das D-1,5-Dihydro-3-phenyl-6-methyl-imidazo[2,1-b]chinazolin-2(3H)-on-hydrochlorid erhalten. Hellgelbe Kristalle vom Schmelzpunkt etwa 320° (Zers.).

### Beispiel 9

In Analogie zu Beispiel 1 wurde aus 2-Amino-benzyl-L-alanin-äthylester das L-1,5-Dihydro-3-methyl-imidazo[2,1-b]chinazolin-2(3H)-on-hydrochlorid erhalten. Braune Kristalle vom Schmelzpunkt 223—226°. Die freie Base schmilzt bei 300—305° unter Zersetzung.

Beispiel 10

In Analogie zu Beispiel 1 wurde aus 2-Amino-benzyl-D-alanin-äthylester das D-1,5-Dihydro-3-methyl-imidazo[2,1-b]chinazolin-2(3H)-on-hydrochlorid erhalten. Gelbe Kristalle vom Schmelzpunkt 225—227°. Die freie Base schmilzt bei etwa 300° unter Zersetzung.

Beispiel 11

Zu einer Lösung von 11,9 g N-(2-Amino-6-chlorbenzyl)-L-alaninäthylester in 20 ml Aethanol wurde bei Zimmertemperatur unter Rühren eine Lösung von 5 g Bromcyan in 20 ml Aethanol zugetropft. Dann wurde das Reaktionsgemisch 1 Stunde am Rückfluss gekocht, zur Trockene eingedampft, der Rückstand mit 150 ml Wasser versetzt und unter Rühren mit 3N $NH_4OH$ alkalisch gestellt. Nach 30 Minute Rühren wurde die Fällung abfiltriert und ans 1N HCl und Acetonitril umkristallisiert. Man erhielt 9,1 g (68% d.Th.) L-6-Chlor-1,5-dihydro-3-methylimidazo[2,1-b]chinazolin-2(3H)-on-hydrochlorid als gelbe Kristalle vom Smp. 260—263°, $[\alpha]_D + 34{,}2°$ (DMSO).

D-6-Chlor-1,5-dihydro-3-methylimidazo[2,1-b]chinazolin-2(3H)-on-hydrochlorid wurde analogerweise aus N-(2-Amino-6-chlorbenzyl)-D-alanin-äthylester erhalten, Smp. 263—266°, $[\alpha]_D - 23{,}9°$ (DMSO); Smp. der freien Base 275—280°.

Das Ausgangsmaterial kann wie folgt hergestellt werden:

18,24 g L-Alaninäthylester-hydrochlorid in 60 ml Aethanol wurde eine Mischung von 25 ml Triäthylamin in 60 ml Aethanol zugetropft und das Gemisch auf 80° erhitzt. Der entstandenen Lösung wurde bei dieser Temperatur eine Lösung von 15 g $\alpha$-Brom-2-chlor-6-nitrotoluol in 60 ml Aethanol zugetropft. Das Gemisch wurde über Nacht bei 80° gerührt und dann zur Trockene eingedampft, der Rückstand mit 150 ml ionenfreiem Wasser versetzt und zweimal mit 100 ml Methylenchlorid extrahiert. Die Methylenchloridextrakte wurden mit Wasser gewaschen, getrocknet und eingedampft. Das so erhaltene Produkt wurde durch Chromatographie an Kieselgel mit Methylenchlorid/5% Methanol gereinigt. Man erhielt 15,75 g (91% d.Th.) N-(2-Chlor-6-nitrobenzyl-L-alaninäthylester, $n_D^{20}$ 1,5267.

N-(2-Chlor-6-nitrobenzyl)-D-alaninäthylester wurde analogerweise aus D-Alaninäthylester und $\alpha$-Brom-2-chlor-6-nitrotoluol erhalten, $n_D^{25}$: 1,5247.

Eine Lösung von 14,3 g N-(2-Chlor-6-nitrobenzyl)-L-alaninäthylester in 50 ml absolutem Aethanol wurde in Gegenwart von 1 g Raney-Nickel hydriert. Nach beendeter Hydrierung wurde vom Katalysator abfiltriert und das Filtrat zur Trockene eingedampft. Man erhielt 12,6 g (99% d.Th.) N-(2-Amino-6-chlorbenzyl)-L-alaninäthylester, $n_D^{22}$ 1,5430.

N-(2-Amino-6-chlorbenzyl)-D-alaninäthylester wurde analogerweise durch Hydrierung von N-(2-Chlor-6-nitrobenzyl)-D-alaninäthylester erhalten, $n_D^{23}$ 1,5405.

Beispiel 12

In zu Beispiel 11 analoger Weise wurden folgende Verbindungen hergestellt:

D-6,7-Dichlor-1,5-dihydro-3-methylimidazo[2,1-b]chinazolin-2(3H)-on-hydrochlorid, Smp. >280°,

L-6,7-Dichlor-1,5-dihydro-3-methylimidazo[2,1-b]chinazolin-2(3H)-on-hydrochlorid, Smp. >290°,

D-7-Brom-1,5-dihydro-3-methylimidazo[2,1-b]chinazolin-2(3H)-on-hydrochlorid, Smp. 268—270°,

L-7-Brom-1,5-dihydro-3-methylimidazo[2,1-b]chinazolin-2(3H)-on-hydrochlorid, Smp. 280—284° (Zers.),

L-6-Chlor-7-methoxy-1,5-dihydro-3-methylimidazo[2,1-b]chinazolin-2(3H)-on-hydrochlorid, Smp. >280°.

Beispiel 13

In zu Beispiel 11 analoger Weise wurden folgende Verbindungen hergestellt:

aus N-(2-Chlor-6-nitrobenzyl)-3-phenyl-D-alanin-äthylester, $[\alpha]_D - 21{,}2°$ (c = 1% in Aethanol) über N-(2-Amino-6-chlorbenzyl)-3-phenyl-D-alanin-äthylester, $[\alpha]_D + 40{,}7°$ (c = 1% in Aethanol), das D-3-Benzyl-6-chlor-1,5-dihydroimidazo[2,1-b]chinazolin-2(3H)-on-hydrochlorid Smp. 260—265° (Zers.); Smp. der Base 270—275° (Zers.);

aus N-(2-Chlor-6-nitrobenzyl)-D-leucin-äthylester über N-(2-Amino-6-chlorbenzyl)-D-leucin-äthylester, $[\alpha]_D + 8{,}5°$ (c = 1% in Aethanol) das D-6-Chlor-1,5-dihydro-3-isobutylimidazo[2,1-b]-chinazolin-2(3H)-on-hydrochlorid, Smp. 290—293°; Smp. der Base 280—285°:

aus N-(2-Chlor-6-nitrobenzyl)-D-serin-äthylester, $[\alpha]_D$ 2,7° (c = 1% in Aethanol), über N-2-(Amino-6-chlorbenzyl)-D-serin-äthylester, Smp. 73—75°, $[\alpha]_D + 65{,}5°$ (c = 1% in Aethanol) das D-6-Chlor-3-hydroxymethyl-1,5-dihydroimidazo[2,1-b]chinazolin-2(3H)-hydrochlorid, Smp. der Base >300° (Zers.);

aus D-N-(2-Chlor-6-nitrobenzyl)-2-phenylglycin-äthylester, $[\alpha]_D - 21°$ (c = 1% in Aethanol), über D-N-(2-Amino-6-chlorbenzyl)-2-phenylglycin-äthylester, $[\alpha]_D - 4{,}5°$ (c = 1% in Aethanol) das D-6-Chlor-3-phenyl-1,5-dihydroimidazo[2,1-b]chinazolin-2(3H)-on-hydrochlorid, Smp. >300° (Zers.); Smp. der Base 260—265° (Zers.).

Beispiel 14

Ein Gemisch von 6 g Aethyl-D-2,5-dichlor-α-methyl-3-(4H)-chinazolin-acetat, 20 ml absolutem Aethanol und 25 ml 5%igem alkoholischem Ammoniak wurde über Nacht im Druckrohr auf 110° erhitzt. Das Druckrohr wurde im Eisbad abgekühlt und geöffnet. Der entstandene Kristallbrei wurde abgenutscht und mit kaltem Aethanol gewaschen. Die erhaltenen Kristalle wurden in 1N Salzsäure gelöst und filtriert. Das Filtrat wurde zur Trockene eingedampft und der Rückstand aus 1N Salzsäure und Acetonitril als D-6-Chlor-1,5-dihydro-3-methyl-imidazo[2,1-b]chinazolin-2(3H)-on-hydrochlorid umkristallisiert (4,3 g, 74% d.Th.), Smp. 275—278°.

Das Ausgangsmaterial kann wie folgt hergestellt werden:

Einem Gemisch von 18, 24 g D-Alaninäthylester-hydrochlorid in 60 ml Aethanol wurde ein Gemisch von 25 ml Triäthylamin in 60 ml Aethanol zugetropft und das Reaktionsgemisch auf 80° erhitzt. Der entstandenen Lösung wurde bei dieser Temperatur eine Lösung von 15 g 2-Chlor-6-nitrobenzylbromid in 60 ml Aethanol zugetropft. Das Gemisch wurde über Nacht bei 80° gerührt, dann zur Trockene eingedampft, der Rückstand mit 150 ml ionenfreiem Wasser versetzt und zweimal mit 100 ml Methylenchlorid extrahiert. Die Methylenchloridextrakte wurden mit Wasser gewaschen, über Natriumsulfat getrocknet und eingedampft. Das so erhaltene Produkte wurde durch Chromatographie an Kieselgel mit Methylenchlorid/5% Methanol gereinigt. Man erhielt 16 g (93% d.Th.) N-(2-Chlor-6-nitrobenzyl)-D-alaninäthylester, $n_D^{25}$ 1,5247; $[\alpha]_D - 6,9°$ (c = 1%, $C_2H_5OH$).

Eine Lösung von 14,3 g N-(2-Chlor-6-nitrobenzyl)-D-alaninäthylester in 50 ml Aethanol wurde in Gegenwart von 1 g Raney-Nickel hydriert. In 2 Stunden wurden 3,35 l Wasserstoff aufgenommen. Dann wurde vom Katalysator abfiltriert und das Filtrat zur Trockene eingedampft. Man erhielt 12,6 g (99% d.Th.) N-(2-Amino-6-chlorbenzyl)-D-alaninäthylester, $n_D^{23}$ 1,5405, $[\alpha]_D + 55,8°$ (c = 1%, $C_2H_5OH$).

Zu einer Lösung von 71,75 g N-(2-Amino-6-chlorbenzyl)-D-alaninäthylester in 400 ml trockenem Tetrahydrofuran wurden unter Stickstoffbegasung und Rühren 52 g N,N'-Carbonyldiimidazol portionsweise zugegeben. Das Gemisch wurde 2 Stunden gerührt und 18 Stunden unter Rückfluss erhitzt und zur Trockene eingedampft und der Rückstand mit 1500 ml Methylenchlorid extrahiert, die organische Phase mit zweimal 400 ml 1N Salzsäure und dann mit 400 ml Wasser gewaschen, getrocknet und eingedampft. Das so erhaltene Oel wurde durch Chromatographie an Kieselgel mit Methylenchlorid/5% Methanol gereinigt. Ausbeute: 79 g (99% d.Th.) Aethyl-D-5-chlor-1,4-dihydro-α-methyl-2-oxo-3(2H)chinazolinacetat, $[\alpha]_D^{25} - 40,8°$ (c = 1%, $C_2H_5OH$).

50,9 g Aethyl-D-5-chlor-1,4-dihydro-α-methyl-2-oxo-3(2H)-chinazolinacetat wurden in 135 ml Phosphoroxychlorid gelöst und 3 Stunden unter Rühren auf 110° erhitzt. Nach dem Kühlen wurde das Reaktionsgemisch zur Trockene eingedampft, der Rückstand in 250 ml Chloroform gelöst, die Lösung mit 300 ml Eiswasser verdünnt und durch tropfenweise Zugabe von 40%igem Natriumhydroxyd bis auf pH 7—8 eingestellt. Die Chloroformphase wurde abgetrennt, getrocknet und eingedampft. Das Produkt wurde durch Chromatographie an Kieselgel mit Methylenchlorid/5% Methanol gereinigt. Ausbeute: 37.4 g (70% d.Th.) Aethyl-D-2,5-dichlor-α-methyl-3(4H)-chinazolinacetat, $n_D^{22}$ 1,5775.

Beispiel 15

Einer Lösung von 5 g D-1,5-Dihydro-3-methylimidazo[2,1-b]chinazolin-2(3H)-on in 80 ml Eisessig Werden tropfenweise 1,5 ml Brom zugesetzt. Das Gemisch wird 1 1/2 Stunden bei Raumtemperaur gerührt, mit 100 ml Wasser verdünnt, auf 30 ml eingeengt, nochmals mit 100 ml Wasser verdünnt, mit 3N Ammoniumhydroxyd alkalisch gestellt, gewaschen und filtriert. Das ausgefallene Produkt wird mit Wasser gewaschen und aus 100 ml 2N HCl umkristallisiert. Man erhält 3,9 g (56%) D-7-Brom-1,5-dihydro-3-methyl-imidazo[2,1-b]chinazolin-2(3H)-on-hydrochlorid, Smp. 268—270°.

Beispiel 16

In zu Beispiel 15 analoger Weise werden 4,7 g L-1,5-Dihydro-3-methylimidazo[2,1-b]chinazolin-2(3H)-on zu 4,2 g (64%) L-7-Brom-1,5-dihydro-3-methyl-imidazo[2,1-b]chinazolin-2(3H)-on-hydrochlorid, Smp. 280—284° (Zers.) bromiert.

Beispiel 17

In üblicher Weise werden Tabletten folgenden Zusammensetzung hergestellt:

| | |
|---|---|
| D-6-Chlor-1,5-dihydro-3-methylimidazo-[2,1-b]chinazolin-2(3H)-on-hydrochlorid | 184,6 mg |
| Milchzucker | 15,0 mg |
| Maisstärke | 37,9 mg |
| Wasserlösliches Polyvinylpyrrolidon | 10,0 mg |
| Magnesiumstearat | 2,5 mg |
| Gesamtgewicht pro Tablette | 250,0 mg |

Beispiel 18

In üblicher Weise werden Gelatinesteckkapseln folgender Zusammensetzung hergestellt:

| | |
|---|---|
| D-6-Chlor-1,5-dihydro-3-methylimidazo-[2,1-b]chinazolin-2(3H)-on-hydrochlorid | 200,0 mg |
| Wasserlösliches Polyvinylpyrrolidon | 2,0 mg |
| Maisstärke | 43,0 mg |
| Talk | 4,5 mg |
| Magnesiumstearat | 0,5 mg |
| Gesamtgewicht pro Kapsel | 250,0 mg |

Beispiel 19

In üblicher Weise wird eine Injektionslösung folgender Zusammensetzung hergestellt:

| | |
|---|---|
| D-6-Chlor-1,5-dihydro-3-methylimidazo-[2,1-b]chinazolin-2(3H)-on-hydrochlorid | 114,16 mg |
| Glycerinformal | 2,4 ml |
| Wasser | 4,0 ml |

**Patentansprüche**

1. Verbindungen der Formel

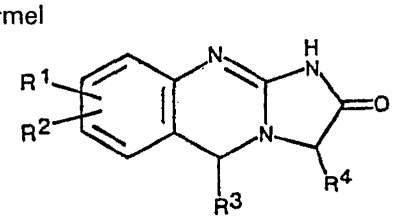

I

worin $R^1$ und $R^2$ Wasserstoff, $C_{1-6}$-Alkyl, Hydroxy, $C_{1-6}$-Alkoxy, Hydroxy-$C_{1-6}$-alkyl, $C_{1-6}$-Alkoxy-$C_{1-6}$-alkyl, Halogen, Phenyl, Phenoxy, Amino, $C_{1-6}$-Alkylamino oder di-$C_{1-6}$-Alkylamino, und $R^1$ und $R^2$ an benachbarten Kohlenstoffatomen auch gemeinsam Methylendioxy; $R^3$ Wasserstoff, $C_{1-6}$-Alkyl oder Phenyl; und $R^4$ $C_{1-6}$-Alkyl, Hydroxy-$C_{1-6}$-alkyl, $C_{1-6}$-Alkoxy-$C_{1-6}$-alkyl, Aryl-$C_{1-6}$-alkyl oder Aryl bedeuten,

und deren Tautomere sowie Salze solcher Verbindungen.

2. D-6-Chlor-1,5-dihydro-3-methylimidazo[2,1-b]chinazolin-2(3H)-on und Salze davon.

3. Eine Verbindung nach Anspruch 1 oder 2 als Mittel zur Hemmung der Blutplättchenaggregation oder zur Verstärkung der Herzkontraktion ohne wesentliche Tachycardie.

4. Verfahren zur Herstellung von Verbindungen der Formel

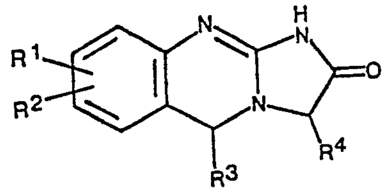

I

worin $R^1$ und $R^2$ Wasserstoff, $C_{1-6}$-Alkyl, Hydroxy, $C_{1-6}$-Alkoxy, Hydroxy-$C_{1-6}$-alkyl, $C_{1-6}$-Alkoxy-$C_{1-6}$-alkyl, Halogen, Phenyl, Phenoxy, Amino, $C_{1-6}$-Alkylamino oder di-$C_{1-6}$-Alkylamino, und $R^1$ und $R^2$ an benachbarten Kohlenstoffatomen auch gemeinsam Methylendioxy; $R^3$ Wasserstoff, $C_{1-6}$-Alkyl oder Phenyl; und $R^4$ $C_{1-6}$-Alkyl, Hydroxy-$C_{1-6}$-alkyl, $C_{1-6}$-Alkoxy-$C_{1-6}$-alkyl, Aryl-$C_{1-6}$-alkyl oder Aryl bedeuten,
und deren Tautomeren sowie von Salzen solcher Verbindungen, dadurch gekennzeichnet, daß man
a) eine Verbindung der Formel

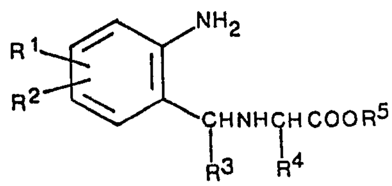

II

worin $R^1$—$R^4$ die obige Bedeutung haben und $R^5$ $C_{1-6}$-Alkyl darstellt,
mit Bromcyan umsetzt, oder
b) eine Verbindung der Formel

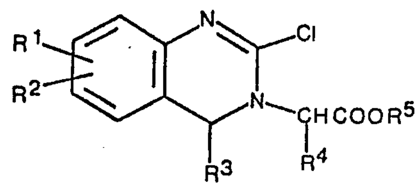

III

worin $R^1$—$R^5$ die obige Bedeutung haben,
mit Ammoniak behandelt.

5. Verfahren nach Anspruch 4 zur Herstellung von D-6-Chlor-1,5-dihydro-3-methylimidazo[2,1-b]chinazolin-2(3H)-on und Salzen davon.

6. Pharmazeutisches Präparat, gekennzeichnet durch einen Gehalt an einer Verbindung der Formel I gemäß Definition in Anspruch 1, einem Tautomeren davon oder einem physiologisch verträglichen Salz einer solchen Verbindung.

7. Verfahren zur Herstellung von pharmazeutischen Präparaten, dadurch gekennzeichnet, daß man eine Verbindung der Formel I gemäß Definition in Anspruch 1, ein Tautomer davon oder ein physiologisch verträgliches Salz einer solchen Verbindung als wirksamen Bestandteil mit zur therapeutischen Verabreichung geeigneten, nicht-toxischen, inerten, an sich in solchen Präparaten üblichen festen und flüssigen Trägern und/oder Excipientien vermischt.

**Revendications**

1. Composés de formule générale I

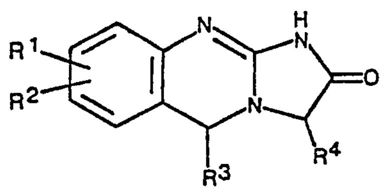

I

dans laquelle:
$R^1$ et $R^2$ représentent un hydrogène, $C_{1-6}$-alkyle, un hydroxy, $C_{1-6}$-alcoxy, hydroxy-$C_{1-6}$-alkyle, $C_{1-6}$-alcoxy-$C_{1-6}$-alkyle, un halogène, phényle, phénoxy, amino, $C_{1-6}$-alkylamino ou di-$C_{1-6}$-alkylamino, et $R^1$ et $R^2$ représentent également ensemble un méthylènedioxy sur deux atomes de carbone voisins; $R^3$ représente un hydrogène, $C_{1-6}$-alkyle ou phényle; et $R^4$ représente $C_{1-6}$-alkyle, hydroxy-$C_{1-6}$-alkyle, $C_{1-6}$-alcoxy-$C_{1-6}$alkyle, aryle-$C_{1-6}$-alkyle ou aryle,
ainsi que leurs tautomères et les sels de ces composés.

2. La D-6-chloro-1,5-dihydro-3-méthylimidazo[2,1-b]quinazolin-2(3H)-one et ses sels.

3. Un composé selon l'une des revendications 1 et 2 en tant qu'agent inhibiteur de l'agrégation des plaquettes sanguines ou en tant qu'agent de renforcement de la contraction cardiaque sans tachycardie notable.

4. Procédé de préparation de composés de formule I

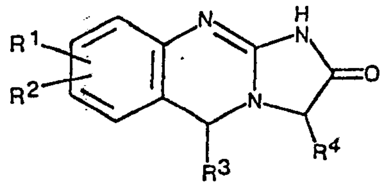

I

dans laquelle:

$R^1$ et $R^2$ représentent un hydrogène, $C_{1-6}$-alkyle, un hydroxy, $C_{1-6}$-alcoxy, hydroxy-$C_{1-6}$-alkyle, $C_{1-6}$-alcoxy-$C_{1-6}$-alkyle, un halogène, un phényle, un phénoxy, un amino, $C_{1-6}$-alkylamino ou di-$C_{1-6}$-alkylamino, et $R^1$ et $R^2$ représentent également ensemble un méthylènedioxy sur deux atomes de carbone voisins; $R^3$ représente un hydrogène, $C_{1-6}$-alkyle ou phényle; et $R^4$ représente $C_{1-6}$-alkyle, hydroxy-$C_{1-6}$-alkyle, $C_{1-6}$-alcoxy-$C_{1-6}$-alkyle, aryle-$C_{1-6}$-alkyle ou aryle,

ainsi que leurs tautomères et les sels de ces composés, caractérisé en ce que:

a) l'on fait réagir un composé de formule

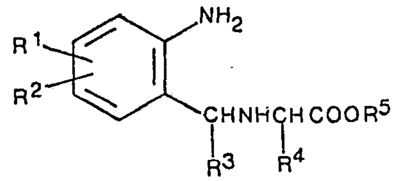

II

dans laquelle:

$R^1$ à $R^4$ ont la signification donnée précédemment et $R^5$ représente $C_{1-6}$-alkyle,

avec du bromocyanure, ou

b) l'on traite un composé de formule

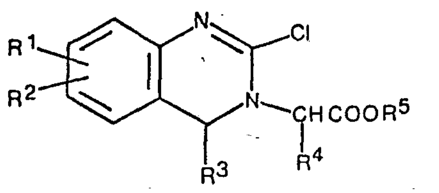

III

dans laquelle:

$R^1$ à $R^5$ ont la signification donnée précédemment, avec de l'ammoniac.

5. Procédé selon la revendication 4, caractérisé en ce que l'on prépare la D-6-chloro-1,5-dihydro-3-méthylimidazo [2,1-b] quinazolin-2(3H)-one et ses sels.

6. Préparation pharmaceutique, caractérisée en ce qu'elle contient un composé de formule générale I selon la définition de la revendication 1, un tautomère ou un sel physiologiquement acceptable d'un tel composé.

7. Procédé de préparation de compositions pharmaceutiques, caractérisé en ce que l'on mélange un composé de formule générale I selon la définition de la revendication 1, un tautomère ou un sel physiologiquement acceptable d'un tel composé, à titre de substance active, avec des supports et/ou excipients liquides ou solides usuels, inertes, non toxiques et appropriés à l'administration thérapeutique.

**Claims**

1. Compounds of the formula

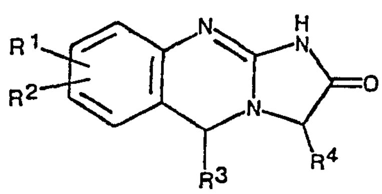

I

wherein $R^1$ und $R^2$ signify hydrogen, $C_{1-6}$-alkyl, hydroxy, $C_{1-6}$-alkoxy, hydroxy-$C_{1-6}$-alkyl, $C_{1-6}$-alkoxy-$C_{1-6}$-alkyl, halogen, phenyl, phenoxy, amino, $C_{1-6}$-alkylamino or di-$C_{1-6}$-alkylamino, and $R^1$

and $R^2$ on adjacent carbon atoms also together signify methylenedioxy; $R^3$ signifies hydrogen, $C_{1-6}$-alkyl, $C_{1-6}$-alkoxy-$C_{1-6}$-alkyl, aryl-$C_{1-6}$-alkyl or aryl,
and their tautomers as well as salts of such compounds.

2. D-6-Chloro-1,5-dihydro-3-methylimidazo[2,1-b]quinazolin-2(3H)-one and salts thereof.

3. A compound according to claim 1 or 2 as an agent for inhibiting blood platelet aggregation or for intensifying the heart contraction without substantial tachycardia.

4. Process for the manufacture of compounds of the formula

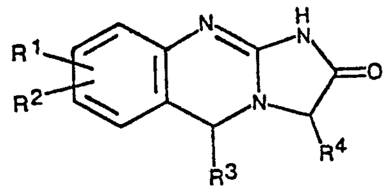

I

wherein $R^1$ und $R^2$ signify hydrogen, $C_{1-6}$-alkyl, hydroxy, $C_{1-6}$-alkoxy, hydroxy-$C_{1-6}$-alkyl, $C_{1-6}$-alkoxy-$C_{1-6}$ alkyl, halogen, phenyl, phenoxy, amino, $C_{1-6}$-alkylamino or di-$C_{1-6}$-alkylamino, and $R^1$ and $R^2$ on adjacent carbon atoms also together signify methylenedioxy; $R^3$ signifies hydrogen, $C_{1-6}$-alkyl or phenyl; and $R^4$ signifies $C_{1-6}$-alkyl, hydroxy-$C_{1-6}$-alkyl, $C_{1-6}$-alkoxy-$C_{1-6}$-alkyl, aryl-$C_{1-6}$-alkyl or aryl,
and their tautomers as well as of salts of such compounds, characterized by
a) reacting a compound of the formula

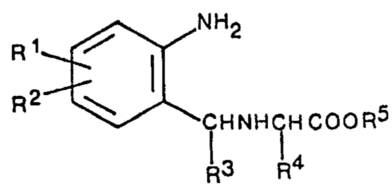

II

wherein $R^1$—$R^4$ have the above significance and $R^5$ represents $C_{1-6}$-alkyl,
with cyanogen bromide, or
b) treating a compound of the formula

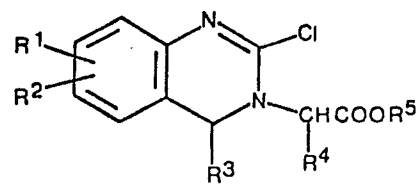

III

wherein $R^1$—$R^5$ have the above significance, with ammonia.

5. Process according to claim 4 for the manufacture of D-6-chloro-1,5-dihydro-3-methylimidazo[2,1-b]quinazolin-2(3H)-one and salts thereof.

6. Pharmaceutical preparation, characterized by a content of a compound of formula I in accordance with the definition in claim 1, a tautomer thereof or a physiologically compatible salt of such a compound.

7. Process for the manufacture of pharmaceutical preparations, characterized by mixing a compound of formula I in accordance with the definition in claim 1, a tautomer thereof or a physiologically compatible salt of such a compound as the active ingredient with non-toxic, inert, solid and liquid carriers which are usual in such preparations and which are suitable for therapeutic administration.